(19) Europäisches Patentamt
European Patent Office
Office européen des brevets

(11) Publication number : 0 642 787 A2

(12) EUROPEAN PATENT APPLICATION

(21) Application number : 94306537.5

(22) Date of filing : 06.09.94

(51) Int. Cl.[6] : A61K 31/00, A61K 31/135, A61K 31/40, A61K 31/445, A61K 31/495

(30) Priority : 09.09.93 US 118557

(43) Date of publication of application : 15.03.95 Bulletin 95/11

(84) Designated Contracting States : AT BE CH DE DK ES FR GB GR IE IT LI LU NL PT SE

(71) Applicant : ELI LILLY AND COMPANY
Lilly Corporate Center
Indianapolis Indiana 46285 (US)

(72) Inventor : Barrett, James Edward
54 Helmlock Drive
North Tarrytown, New York 10591 (US)
Inventor : Helton, David Reed
2607 South Bo-Mar Lane
Greenfield, Indiana 46140 (US)
Inventor : Rasmussen, Kurt
9735 Logan Lane
Fishers, Indiana 46038 (US)

(74) Representative : Tapping, Kenneth George et al
Lilly Industries Limited
European Patent Operations
Erl Wood Manor
Windlesham Surrey GU20 6PH (GB)

(54) **Cessation of tobacco use.**

(57) Pharmaceuticals which are $5HT_{1A}$ antagonists alleviate the symptoms of withdrawal from tobacco or nicotine and assist patients to withdraw from such use.

EP 0 642 787 A2

The present invention belongs to the fields of pharmacology and pharmaceutical chemistry, and provides a new method of assisting people who are dependent on the use of tobacco or nicotine to break the habit.

It is well known that the chronic administration of nicotine results in tolerance and, eventually, dependence. The use of tobacco has become extremely widespread in all countries, despite the well known adverse effects of the use of tobacco in all its forms. Thus, it is clear that tobacco use is extremely habit-forming, if not addictive, and that its use provides sensations to the user which are pleasant and welcome, even though the user is fully aware of the drastic long term ill effects of its use.

Rather recently, vigorous campaigns against the use of tobacco have taken place, and it is now common knowledge that the cessation of smoking brings with it numerous unpleasant withdrawal symptoms, which include irritability, anxiety, restlessness, lack of concentration, lightheadedness, insomnia, tremor, increased hunger and weight gain, and, of course, a craving for tobacco.

At the present time, probably the most widely used therapy to assist the cessation of tobacco use is nicotine replacement, by the use of nicotine chewing gum or nicotine-providing transdermal patches. It is widely known, however, that nicotine replacement is not effective without habit-modifying psychological treatment and training.

A few drugs have been used to treat nicotine dependence, including clonidine, an α-noradrenergic agonist, and alprazolam, a benzodiazepine agonist. A few of the benzodiazepine psychotropic drugs have been described as of some use in tobacco cessation but are not in widespread use. Some serotonin affecting drugs have been described as anti-smoking aids, particularly including buspirone, which has been described by West et al. as a promising aid to people trying to cease the use of tobacco. Psychopharmacology 104:91-96 (1991). Buspirone is a 5-$HT_{1A}$ (serotonin 1A receptor) partial agonist, with considerably higher agonist activity than antagonist activity. However, 5-$HT_{1A}$ antagonists have not previously been discovered to be useful in the cessation of smoking or tobacco use in general. Such antagonists have been indicated as useful for many purposes in the literature, including depression, appetite disorders, anxiety, sexual dysfunction, various gastrointestinal disorders, headaches and cardiovascular disorders. The literature does not teach a connection between any of such disorders and the cessation of tobacco use.

The present invention, however, is based on the discovery that 5-$HT_{1A}$ antagonists are unexpectedly effective in assisting in the cessation of tobacco use.

The present invention provides a method of alleviating the symptoms caused by withdrawal or partial withdrawal from the use of tobacco or of nicotine which comprises the administration to a patient in need of such treatment of a pharmaceutical which is an antagonist of the 5-$HT_{1A}$ receptor. The invention also provides a method of assisting a person who uses tobacco or nicotine to cease or reduce such use, as well as a method of preventing a person who has ceased or reduced the use of tobacco or nicotine from resuming the use thereof, comprising the administration to such a person of a pharmaceutical which is an antagonist of the 5-$HT_{1A}$ receptor.

Known 5-$HT_{1A}$ antagonists useful for that purpose are spiperone, 8-[4-(4-fluorophenyl)-4-oxobutyl]-1-phenyl-1,3,8-triazaspiro[4.5]decan-4-one; ((S)-UH-301 ((S)-5-fluoro-8-hydroxy-2-(dipropylamino)tetralin); and WAY100135 (N-t-butyl-3-[4-(2-methoxyphenyl)-piperazin-1-yl]-2-phenylpropanamide).

A further group of 5-$HT_{1A}$ antagonists which are particularly preferred for use in the method of the present invention are those described by the formula

$$\text{Ar-O-}\underbrace{\text{CH}_2\overset{\overset{\text{OH}}{|}}{\text{C}}\text{HCH}_2}_{R_1}\text{NHZ} \qquad \text{I}$$

wherein Ar is

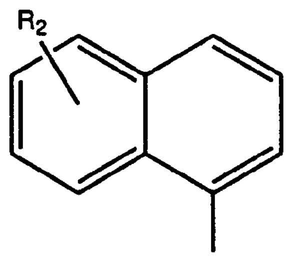

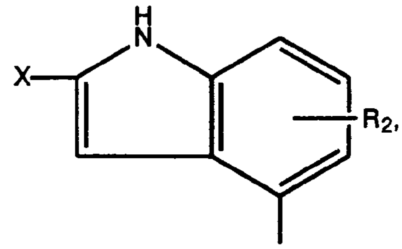

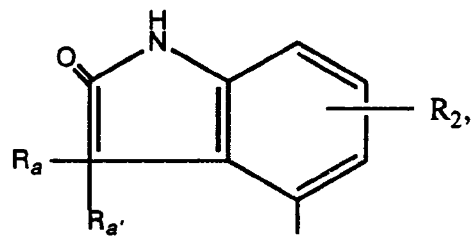

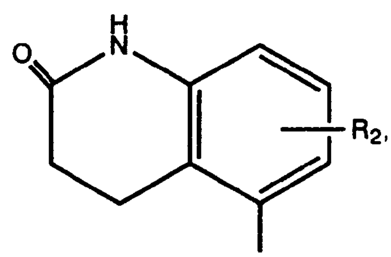

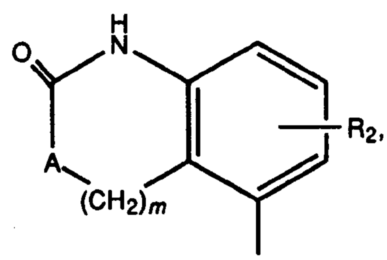

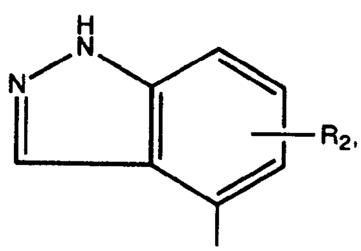

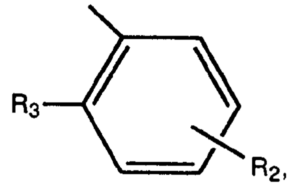

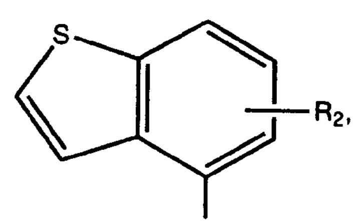

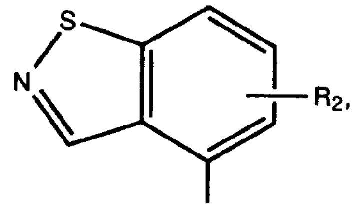

or

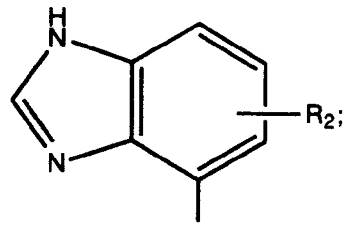

$R_1$ is an optional methyl group substituted on one of the three connecting carbon atoms;

$R_2$ is hydrogen, $C_1$-$C_4$ alkyl, trifluoromethyl, hydroxy, ($C_1$-$C_4$ alkyl)-O-, ($C_1$-$C_4$ alkyl)-S(O)$_p$-, or halo;

$R_3$ is $C_3$-$C_8$ cycloalkyl or a bicycloalkyl group of the formula

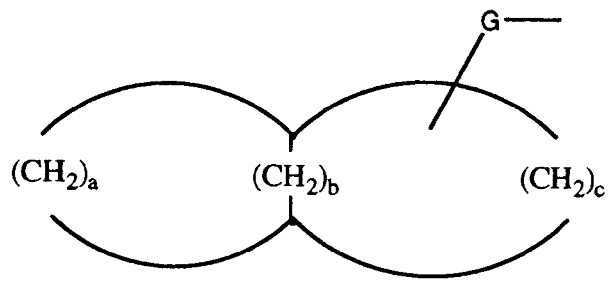

where a and c are independently 1-5, b is 0-5, and (a+c) is greater than 2;

Z is a straight or branched $C_4$-$C_{10}$ alkane, alkene, or alkyne group; ($C_4$-$C_8$ cycloalkyl) optionally substituted with $C_1$-$C_4$ alkyl or phenyl; a bicycloalkyl group of the formula

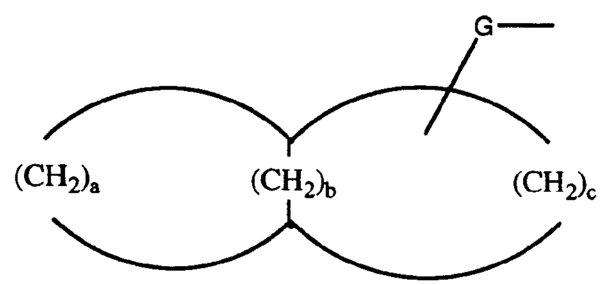

wherein a and c are independently 1-5, b is 0-5, and (a+c) is greater than 2; optionally phenyl substituted $C_2$-$C_{10}$ alkyl where the phenyl group can be optionally substituted with $R_2$ as previously defined; or ($C_1$-$C_4$ alkylidene)-T-($C_1$-$C_4$ alkyl), where T is -O-, -S-, -SO-, or -$SO_2$-;

where

each G is independently a bond or $C_1$-$C_4$ alkylidene;

X is -H, -COY, -CN, or $C_1$-$C_4$ alkyl;

Y is -OH, -O-($C_1$-$C_4$ alkyl), or -$NH_2$;

$R_a$ and $R_{a'}$ are independently hydrogen or $C_1$-$C_3$ alkyl, or when taken together with the carbon atom to which they are attached form a $C_3$-$C_8$ cycloalkyl ring;

p is 0, 1, or 2;
A is -O-, -S-, -NH-, or $-NCH_3-$; and
m is 0, 1, 2, or 3; or a pharmaceutically acceptable salt thereof.

## THE COMPOUNDS

Spiperone is a well-known compound, taught in U.S. Patents 3,155,669, 3,155,670 and 3,161,644. Its activity as a $5-HT_{1A}$ antagonist is shown by, among other articles, Middlemiss et al., Neurosci. and Biobehav. Rev. 16, 75-82 (1992).

(S)-UH-301 is also known to pharmacologists and pharmaceutical chemists. Hillver et al. taught its synthesis in J. Med. Chem. 33, 1541-44 (1990), and Moreau et al., Brain Res. Bull. 29, 901-04 (1992) provided considerable in vivo data about the compound and confirmed that it is a $5-HT_{1A}$ antagonist.

WAY100135 was disclosed by Abou-Gharbia et al., U.S. Patent 4,988,814, who taught that the compound has affinity for the $5-HT_{1A}$ receptor, and can displace 8-hydroxy-2-dipropylaminotetralin from that receptor. Cliffe, et al., J. Med. Chem. 36, 1509-10 (1993) demonstrate that the compound is a $5HT_{1A}$ antagonist with no $5HT_{1A}$ agonist activity.

The compounds of formula I are taught by Beedle, et al., U.S. patent 5,013,761, the description of which is incorporated herein by reference. The synthesis and characteristics, including the $5HT_{1A}$ antagonist activity, of the compounds is shown in that patent.

The particularly preferred compounds of formula I include, for example, the following individual compounds. It will be understood that the following compounds are typical of those of formula 1 but that the compounds include numerous other valuable species as shown by the previously mentioned U.S. patent. It will be further understood that, while individual salts, and in some cases, enantiomers, are mentioned below and are of particular interest, other salts, and enantiomers, diastereomers, and racemates, are likewise valuable and are included in formula I as agents for the present invention.

1-(4-indolyloxy)-3-cyclohexylamino-2-propanol, maleate salt;
cis-1-(4-indolyloxy)-3-(4-phenylcyclohexylamino)-2-propanol, oxalate salt;
1-(4-indolyloxy)-3-(2-phenylethylamino)-2-propanol, oxalate salt;
1-(4-indolyloxy)-3-(3-phenylpropylamino)-2-propanol, oxalate salt;
1-(4-indolyloxy)-3-(4-phenylbutylamino)-2-propanol, oxalate salt;
1-(4-indolyloxy)-3-cyclopentylamino-2-propanol, maleate salt;
1-(4-indolyloxy)-3-cycloheptylamino-2-propanol; (S)-(-)-1-(4-indolyloxy)-3-cyclohexylamino-2-propanol, maleate salt;
(+)-1-(4-indolyloxy)-3-cyclohexylamino-2-propanol, maleate salt;
1-(4-indolyloxy)-3-(3-methylcyclohexylamino)-2-propanol;
1-(4-indolyloxy)-3-(4-methylcyclohexylamino)-2-propanol;
1-(4-indolyloxy)-3-(5-phenylpentylamino)-2-propanol, oxalate salt;
1-(4-indolyloxy)-3-(6-phenylhexylamino)-2-propanol, oxalate salt;
1-(4-indolyloxy)-3-(2,3-dimethylcyclohexylamino)-2-propanol, oxalate salt;
(+-)-1-(4-indolyloxy)-3-(3-pentylamino)-2-propanol;
(R)-(+)-1-(4-indolyloxy)-3-cyclohexylamino)-2-propanol, butanedioate salt;
(R)-(-)-1-(4-indolyloxy)-3-cyclohexylamino)-2-propanol, butanedioate salt;
1-(2-trifluoromethyl-4-benzimidazolyl)-3-(4-phenylbutylamino)-2-propanol;
(exo)-1-(4-indolyloxy)-3-(norbornylamino)-2-propanol;
(endo)-1-(4-indolyloxy)-3-(norbornylamino)-2-propanol;
1-(1-napthalenyloxy)-3-cycloheptylamino-2-propanol, oxalate salt;
1-(2-cyclopentylphenoxy)-3-cycloheptylamino-2-propanol, oxalate salt;
1-(2-cyclohexylphenoxy)-3-cyclooctylamino-2-propanol, oxalate salt;
1-(2-cycloheptylphenoxy)-3-(1,2,3-trimethyl-2-propylamino)-2-propanol, oxalate salt; and
1-(2-cyclopropylphenoxy)-3-(1,1-dimethylbutylamino)-2-propanol, oxalate salt.

The group of the compounds of formula I wherein the group Ar is indolyl or substituted indolyl constitutes a further preferred class of $5-HT_{1A}$ antagonists; and the compounds of formula 1 wherein Z is ($C_4$-$C_8$ cycloalkyl) optionally substituted with $C_1$-$C_4$ alkyl or phenyl; or Z represents optionally phenyl substituted $C_2$-$C_{10}$ alkyl where the phenyl group can be optionally substituted with $R_2$; constitute further particularly preferred classes of compounds for use in the present invention.

Still further, compounds which are full $5-HT_{1A}$ antagonists, having substantially no $5-HT_{1A}$ agonist activity, are particularly preferred for use in the present invention. It will be understood that biologically active compounds very often combine more than one activity, and particularly that compounds may be and frequently

are both antagonists and agonists at the same receptor, in various degrees. Buspirone, for example, is a partial agonist having about 70% agonistic and about 30% antagonistic at the $5HT_{1A}$ receptor. In the present document, the term "$5HT_{1A}$ antagonist" means a substance which is fully or substantially fully antagonistic, with no agonist activity or only a negligible amount of agonist activity, compared to its antagonist activity. For example, a compound which has 80% antagonist and 20% agonist activity at the $5HT_{1A}$ receptor would be considered an antagonist for the purposes of the present invention.

## THE CONDITION AND TREATMENT METHODS

The method of the present invention is broadly useful in assisting persons who want to cease or reduce their use of tobacco or nicotine. Most commonly, the form of tobacco use is smoking, most commonly the smoking of cigarettes. The present invention is also helpful, however, in assisting in breaking the habit of all types of tobacco smoking, as well as the use of snuff, chewing tobacco, etc. The present method is also helpful to those who have replaced, or partially replaced, their use of tobacco with the use of nicotine replacement therapy. Thus, such patients can be assisted to reduce and even eliminate entirely their dependence on nicotine in all forms.

It will be understood that the present invention is useful for preventing or alleviating the withdrawal symptoms which afflict patients who are trying to eliminate or reduce their use of tobacco or nicotine. The common withdrawal symptoms of such people include, at least, irritability, anxiety, restlessness, lack of concentration, insomnia, nervous tremor, increased hunger and weight gain, light-headedness, and the craving for tobacco or nicotine. The prevention or alleviation of such symptoms, when they are caused by or occur in conjunction with ceasing or reducing the patient's use of tobacco or nicotine is a desired result of the present invention and an important aspect of it.

The present invention is carried out by administering a compound described above, either spiperone or a compound of formula I, to a patient who is in need of or carrying out a reduction or cessation of tobacco or nicotine use.

The dose of compound to be administered, in general, is from about 1 to about 100 mg/day; as usual, the daily dose may be administered in a single bolus, or in divided doses, depending on the judgment of the physician in charge of the case. A more preferred range of doses is from about 5 to about 100 mg/day; other dosage ranges which may be preferred in certain circumstances are from about 10 to about 50 mg/day; from about 5 to about 50 mg/day; from about 10 to about 25 mg/day; and a particularly preferred range is from about 20 to about 25 mg/day. It will be understood that the dose for a given patient is always to be set by the judgment of the attending physician, and that the dose is subject to modification based on the size of the patient, the lean or fat nature of the patient, the characteristics of the particular compound chosen, the intensity of the patient's tobacco habit, the intensity of the patient's withdrawal symptoms, and psychological factors which may affect the patient's physiological responses.

## THE FORMULATIONS

Pharmaceuticals are substantially always formulated into pharmaceutical dosage forms, in order to provide an easily controllable dosage of the drug, and to give the patient an elegant and easily handled product. The present compounds are susceptible to formulation into the conventional pharmaceutical dosage forms, including capsules, tablets, inhalants, injectable parenteral solutions and suppositories. Usually the oral dosage forms, particularly tablets and capsules, are most convenient for the patient and are usually preferred. Liquid suspensions of pharmaceuticals, formerly popular, have now become less popular and are seldom used but the present compounds are entirely amenable to such products should they be desired.

While it is possible to administer a compound of the invention directly, the compounds are preferably employed in the form of a pharmaceutical formulation comprising a pharmaceutically acceptable carrier, diluent or excipient and a compound of the invention. Such formulations will contain from about 0.01 percent to about 90 percent of a compound of the invention.

In making the formulations of the present invention, the active ingredient will usually be mixed with at least one carrier, or diluted by at least one carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the formulations can be in the form of tablets, granules, pills, powders, lozenges, sachets, cachets, elixirs, emulsions, solutions, syrups, suspensions, aerosols (as a solid or in a liquid medium) and soft and hard gelatin capsules.

Examples of suitable carriers, diluents and excipients include lactose, dextrose, sucrose, sorbitol, mannitol, starches, gum acacia, calcium phosphate, alginates, liquid paraffin, calcium silicate, microcrystalline cellulose, polyvinylpyrrolidone, cellulose, tragacanth, gelatin, syrup, methylcellulose, methyl- and propyl-hydroxybenzoates, vegetable oils, such as olive oil, injectable organic esters such as ethyl oleate, talc, magnesium stearate, water and mineral oil. The formulations may also include wetting agents, lubricating, emulsifying and suspending agents, preserving agents, sweetening agents, perfuming agents, stabilizing agents or flavoring agents. The formulations of the invention may be formulated so as to provide quick, sustained or delayed release of the active ingredient after administration to the patient by employing procedures well-known in the art.

For oral administration, a compound of this invention ideally can be admixed with carriers and diluents and molded into tablets or enclosed in gelatin capsules.

The compositions are preferably formulated in a unit dosage form, each unit containing from about 1 to about 100 mg, more usually about 5 to about 100 mg, of the active ingredient. The term "unit dosage form" refers to physically discrete units suitable as unitary dosages for human subjects and other mammals, each unit containing a predetermined quantity of active material calculated to produce the desired therapeutic effect, related to the desired daily or divided dose, in association with a suitable pharmaceutical carrier, diluent or excipient therefor.

In order to more fully illustrate the operation of this invention, the following examples of formulations are provided. The examples are illustrative only and are not intended to limit the scope of the invention. The formulations may employ as active compounds any of the compounds of the present invention.

## FORMULATION 1

Hard gelatin capsules are prepared using the following ingredients:

| | Amt. per Capsule | Concentration by Weight (percent) |
|---|---|---|
| 1-(4-indolyloxy)-3-cyclopentylamino-2-propanol, hydrochloride salt | 250 mg | 55.0 |
| Starch dried | 220 mg | 43.0 |
| Magnesium stearate | 10 mg | 2.0 |
| | 460 mg | 100.0 |

The above ingredients are mixed and filled into hard gelatin capsules in 460 mg quantities.

## FORMULATION 2

Capsules each containing 20 mg of medicament are made as follows:

| | Amt. per Capsule | Concentration by Weight (percent) |
|---|---|---|
| 1-(4-indolyloxy)-3-(5-phenylpentylamino)-2-propanol, maleate salt | 20 mg | 10.0 |
| Starch | 89 mg | 44.5 |
| Microcrystalline cellulose | 89 mg | 44.5 |
| Magnesium stearate | 2 mg | 1.0 |
| | 200 mg | 100.0 |

The active ingredient, cellulose, starch and magnesium stearate are blended, passed through a No. 45 mesh U.S. sieve and filled into a hard gelatin capsule.

## FORMULATION 3

Capsules each containing 100 mg of active ingredient are made as follows:

| | Amt. per Capsule | Concentration by Weight (percent) |
|---|---|---|
| 1-(1-naphthalenyloxy)-3-cyclopentylamino-2-propanol, hydrochloride salt | 100 mg | 29.0 |
| Polyoxyethylenesorbitan monooleate | 50 mcg | 0.02 |
| Starch powder | 250 mg | 71.0 |
| | 250.05 mg | 100.02 |

The above ingredients are thoroughly mixed and placed in an empty gelatin capsule.

**FORMULATION 4**

Tablets each containing 10 mg of active ingredient are made up as follows:

| | Amt. per Capsule | Concentration by Weight (percent) |
|---|---|---|
| 1-(2-cyclohexylphenoxy)-3-cyclopentylamino-2-propanol, oxalate salt | 10 mg | 10.0 |
| Starch | 45 mg | 45.0 |
| Microcrystalline cellulose | 35 mg | 35.0 |
| Polyvinyl pyrrolidone (as 10% solution in water) | 4 mg | 4.0 |
| Sodium carboxyethyl starch | 4.5 mg | 4.5 |
| Magnesium stearate | 0.5 mg | 0.5 |
| Talc | 1 mg | 1.0 |
| | 100 mg | 100.0 |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granule so produced is dried at 50°-60°C and passed through a No. 18 mesh U.S. sieve. The sodium carboxymethyl starch, magnesium stearate and talc, previously passed through a No. 60 mesh U.S. sieve, are then added to the granule which, after mixing, is compressed on a tablet machine to yield a tablet weighing 100 mg.

**FORMULATION 5**

A tablet formula may be prepared using the ingredients below:

| | Amt. per Capsule | Concentration by Weight (percent) |
|---|---|---|
| 1-(2-cyclopropylphenoxy)-3-(1,1-dimethylethylamino)-2-propanol, maleate salt | 250 mg | 38.0 |
| Cellulose microcrystalline | 400 mg | 60.0 |
| Silicone dioxide fumed | 10 mg | 1.5 |
| Stearic acid | 5 mg | 0.5 |
| | 665 mg | 100.0 |

The components are blended and compressed to tablets each weighing 665 mg.

## FORMULATION 6

Suspensions each containing 5 mg of medicamen 40 ml dose are made as follows:

| | Per 5 ml of suspension |
|---|---|
| (exo)-1-(4-indolyloxy)-3-norbornylamino-2-propanol | 5 mg |
| Sodium carboxymethyl cellulose | 50 mg |
| Syrup | 1.25 ml |
| Benzoic acid solution | 0.10 ml |
| Flavor | q.v. |
| Color | q.v. |
| Water | q.s. to 5 ml |

The medicament is passed through a No. 45 mesh U.S. sieve and mixed with the sodium carboxymethylcellulose and syrup to form a smooth paste. The benzoic acid solution, flavor and color is diluted with some of the water and added, with stirring. Sufficient water is then added to produce the required volume.

## THE TESTING METHODS

Pharmacologists experienced in working with the central nervous system are aware that $5HT_{1A}$ antagonists may be identified by a conventional test in laboratory animals, which is carried out as follows.

### Serotonin Syndrome Tests

Adult male rats are placed singly in clear plastic cages for behavioral observations. Preferably, the cages have metal screen lids and fresh sawdust covering the floor. The animals are adapted to the cages for a short period, and then the compounds to be tested are administered at doses and route determined by the experimenter, based on the probable potency of the test compounds. After 15 minutes, each rat is then given a subcutaneous injection of 0.1 mg/kg of 8-hydroxy-2-dipropylaminotetralin (8-OH-DPAT). Fifteen minutes after administration of the 8-OH-DPAT, the animals are evaluated for evidence of the following signs: reciprocal forepaw treading; Straub tail (rigid or corkscrew); lateral head weaving; hind limb abduction; resting tremor, especially of the head and forepaws; lower-lip retraction; and flat-body posture.

Those signs are produced by 8-OH-DPAT, and the administration of $5HT_{1A}$ antagonists is able to block or greatly attenuate the appearance of the signs. Conventionally, the severity of the above signs is assessed on a 4 point scale, where 0 indicates the absence of the sign and 3 indicates that it is present in a marked degree. Tests of representative compounds of formula I were carried out according to the above test method, administering the test compounds subcutaneously, with the following results.

### Antagonism Test Results

The compound of formula I, (S)-(-)-1-(4-indolyloxy)-3-cyclohexylamino-2-propanol, butanedioate salt, was tested against 8-OH-DPAT, as described above. Doses of the test compound from 0.01 mg/kg to 3 mg/kg were administered. In the control experiments, with no test compound, scores of 3 for flat body posture (FBP) and for lower lip retraction (LLR) were recorded. The administration of 3 mg/kg of the test compound completely abolished both signs in the test animals; and the administration of 0.3 mg/kg reduced the scores below 2 on both signs. Thus, it is clear that the compound is an effective $5HT_{1A}$ antagonist.

Another compound, (exo)-1-(4-indolyloxy)-3-norbornylamino-2-propanol, was tested in the same manner at doses from 0.01 mg/kg to 10 mg/kg. Again, scores of 3 for both the FBP and LLR signs were observed when 8-OH-DPAT was administered alone. Both the 1.0 mg/kg and 10 mg/kg doses of the test compound reduced the FBP score to 0.5 or below, and reduced the LLR score to 1 or below, except for a score of 1.25 at 20 minutes after administration of 8-OH-DPAT. This compound, also, is seen to be an effective $5HT_{1A}$ antagonist.

The compound 1-(4-indolyloxy)-3-(4-phenylbutylamino)-2-propanol, oxalate salt, was administered in the same test method at doses of 1 and 10 mg/kg. The usual scores of 3 were observed in the animals to which only 8-OH-DPAT was administered. Administration at 10 mg/kg gave an FBP score of 1.25, and a peak LLR score of 2; the LLR score, however, quickly declined from its peak of 2. The compound is shown to be a $5HT_{1A}$ antagonist.

Another compound, 1-(4-indolyloxy)-3-cycloheptylamino-2-propanol, was tested at doses from 0.1 mg/kg to 10 mg/kg, in a series of tests where 8-OH-DPAT provided the usual scores of 3 in FBP and LLR. Doses of 1 mg/kg and above reduced the FBP to below 1. The LLR score was reduced to 0 by the 10 mg/kg dose, while 1 and 3 mg/kg doses gave scores of 1.75 or less.

The above results demonstrate the convenience of the serotonin syndrome test for the identification of $5HT_{1A}$ antagonists, and show the effect of representative compounds of formula I

Nicotine Withdrawal Evaluation by the Startle Test

The effect of $5HT_{1A}$ antagonists in alleviating the symptoms of nicotine withdrawal was evaluated in rats by an auditory startle test, which was carried out as follows.

Procedures for Nicotine Withdrawal Studies

Animals: Male Long Evans rats were individually housed in a controlled environment on a 12 hour light-dark cycle and were given free access to food (Purina Rodent Chow) and water. All treatment groups contained 8-10 rats.

Chronic Nicotine Treatment: Rats were anesthetized with halothane and Alzet osmotic minipumps (Alza Corporation, Palo Alto, CA, Model 2ML2) were implanted subcutaneously. Nicotine ditartrate was dissolved in physiological saline. Pumps were filled with either nicotine ditartrate (6 mg/kg base/day) or physiological saline. Twelve days following implantation of pumps, rats were anesthetized with halothane and the pumps were removed.

Auditory Startle Respose: The sensory motor reactions [auditory startle response (peak amplitude Vmax)] of individual rats was recorded using San Diego Instruments startle chamers (San Diego, CA). Startle sessions consisted of a 5-minute adaptation period at a background noise level of 70±3 dBA immediately followed by 25 resentations of auditory stimuli (120±2 dBA noise, 50 ms duration) presented at 8-second intervals. Peak startle mplitudes were then averaged for all 25 presentations of timuli for each session. Auditory startle responding was evaluated daily at 24 hour intervals on days 1-4 following nicotine withdrawal.

Results: As illustrated in Tables 1, 2, and 3 subcutaneous administration of representative compounds of Formula I 15 minutes prior to startle testing significantly attenuated the effects of nicotine withdrawal on the auditory startle reflex.

The compounds were as follows:

A. 1-(4-indolyloxy)-3-cyclohexylamino-2-propanol, butanedioate salt.

B. (S)-(-)-(4-indolyloxy)-3-cyclohexylamino-2-propanol, butanedioate salt

C. 1-(4-indolyloxy)-3-(3-methylcyclohexylamino)-2-propanol

**Table 1**

| Chronic Treatment | Pre-Treatment | Startle Response (μV) | | | |
|---|---|---|---|---|---|
| | | Day 1 | Day 2 | Day 3 | Day 4 |
| Saline | Saline | 265±19 | 308±22 | 294±21 | 305±25 |
| Nicotine | Saline | 372±24* | 366±22* | 439±28* | 421±26* |
| Nicotine | Compound A 1.0 mg/kg | 306±17 | 332±15* | 342±21* | 343±17 |
| Nicotine | Compound A 3.0 mg/kg | 265±20 | 319±21 | 279±18 | 314±20 |
| Nicotine | Compound A 10 mg/kg | 277±17 | 315±18 | 294±18 | 253±17 |

* Significantly different from Saline/Saline control, $P<0.05$

**Table 2**

| Chronic Treatment | Pre-Treatment | Startle Response (μV) | | | |
|---|---|---|---|---|---|
| | | Day 1 | Day 2 | Day 3 | Day 4 |
| Saline | Saline | 289±17 | 287±20 | 270±21 | 275±16 |
| Nicotine | Saline | 403±29* | 357±32* | 383±32* | 354±28* |
| Nicotine | Compound B 3.0 mg/kg | 218±10 | 250±17 | 250±17 | 287±23 |

* Significantly different from Saline/Saline control, $P<0.05$

**Table 3**

| Chronic Treatment | Pre-Treatment | Startle Response (μV) | | | |
|---|---|---|---|---|---|
| | | Day 1 | Day 2 | Day 3 | Day 5 |
| Saline | Saline | 284±18 | 272±18 | 230±24 | 249±19 |
| Nicotine | Saline | 345±25* | 384±24* | 350±26* | 334±25* |
| Nicotine | Compound C 0.1 mg/kg | | | 307±19* | 330±29* |
| Nicotine | Compound C 1.0 mg/kg | 280±18 | 299±20 | 255±17 | 228±17 |
| Nicotine | Compound C 10 mg/kg | 240±18 | 195±15 | | |

* Significantly different from Saline/Saline control, $P<0.05$

## Claims

1. Use of a pharmaceutical which is an antagonist of the $5HT_{1A}$ receptor for the manufacture of a medicament for alleviating the symptoms caused by withdrawal or partial withdrawal from the use of tobacco or of nicotine.

2. A use of **Claim 1** wherein the withdrawal symptom is irritability, anxiety, restlessness, lack of concentration, light headedness, insomnia, tremor, increased hunger or weight gain.

3. A use of **Claim 1** wherein the symptoms are caused by withdrawal or partial withdrawal from the use of tobacco.

4. A use of **Claim 1** for assisting the patient to cease or reduce the use of tobacco or nicotine.

5. A use of **Claim 1** wherein the $5HT_{1A}$ antagonist is spiperone, (S)-UH-301, or WAY100135.

6. A method of **Claim 1** wherein the $5HT_{1A}$ antagonist is a compound of the formula

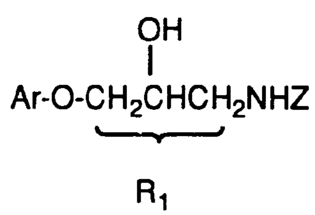

I

wherein Ar is

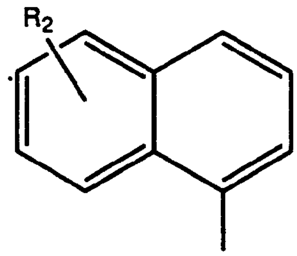

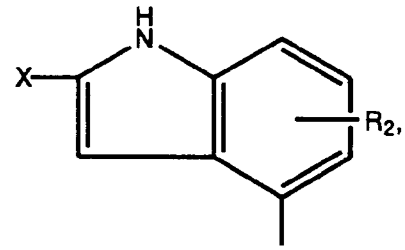

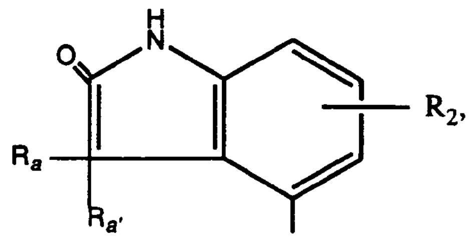

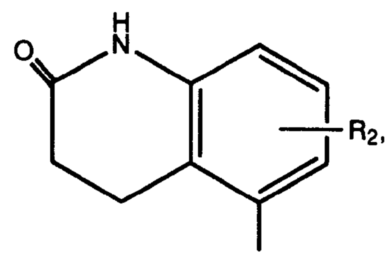

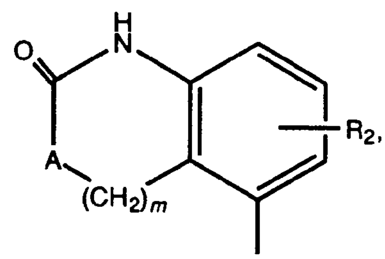

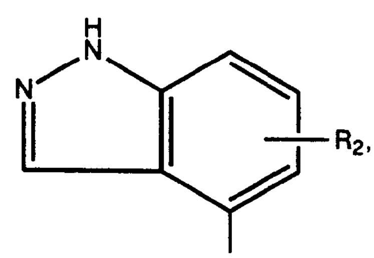

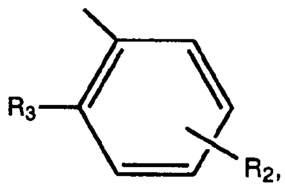

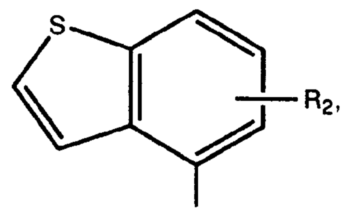

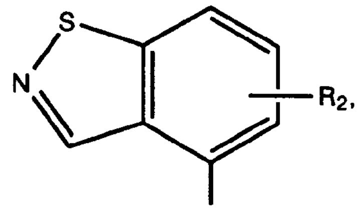

or

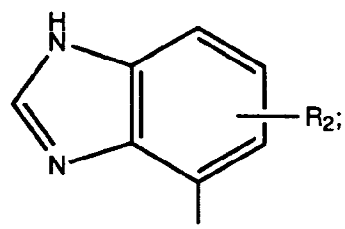

$R_1$ is an optional methyl group substituted on one of the three connecting carbon atoms;
$R_2$ is hydrogen, $C_1$-$C_4$ alkyl, trifluoromethyl, hydroxy, ($C_1$-$C_4$ alkyl)-O-, ($C_1$-$C_4$ alkyl)-$S(O)_p$-, or halo;
$R_3$ is $C_3$-$C_8$ cycloalkyl or a bicycloalkyl group of the formula

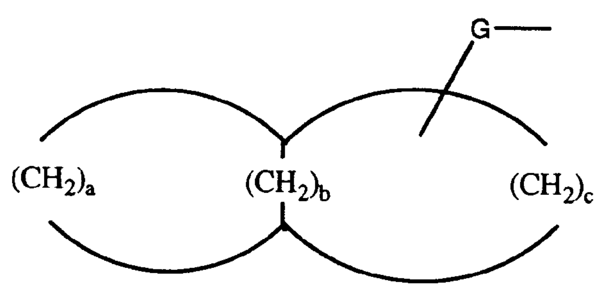

where a and c are independently 1-5, b is 0-5, and (a+c) is greater than 2;

Z is a straight or branched $C_4$-$C_{10}$ alkane, alkene, or alkyne group; ($C_4$-$C_8$ cycloalkyl) optionally substituted with $C_1$-$C_4$ alkyl or phenyl; a bicycloalkyl group of the formula

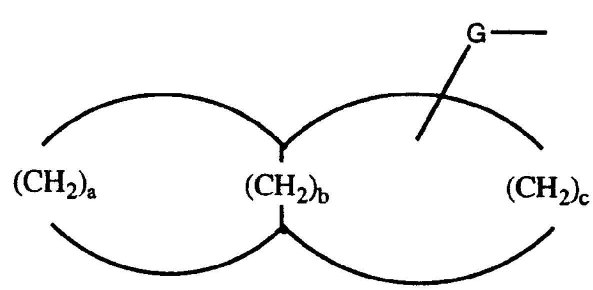

wherein a and c are independently 1-5, b is 0-5, and (a+c) is greater than 2; optionally phenyl substituted $C_2$-$C_{10}$ alkyl where the phenyl group can be optionally substituted with $R_2$ as previously defined; or ($C_1$-$C_4$ alkylidene)-T-($C_1$-$C_4$ alkyl), where T is -O-, -S-, -SO-, or -$SO_2$-;
where

each G is independently a bond or $C_1$-$C_4$ alkylidene;
X is -H, -COY, -CN, or $C_1$-$C_4$ alkyl;
Y is -OH, -O-($C_1$-$C_4$ alkyl), or -$NH_2$;
$R_a$ and $R_{a'}$ are independently hydrogen or $C_1$-$C_3$ alkyl, or when taken together with the carbon atom to which they are attached form a $C_3$-$C_8$ cycloalkyl ring;
p is 0, 1, or 2;
A is -O-, -S-, -NH-, or -$NCH_3$-; and
m is 0, 1, 2, or 3; or a pharmaceutically acceptable salt thereof.

7. A use of **Claim 6** wherein the $5HT_{1A}$ antagonist is a compound wherein the group Ar is indolyl or substituted indolyl.

8. A use of **Claim 6** wherein the group Z is ($C_4$-$C_8$ cycloalkyl) optionally substituted with $C_1$-$C_4$ alkyl or phenyl.

9. A use of **Claim 6** wherein the group Z is optionally phenyl substituted $C_2$-$C_{10}$ alkyl where the phenyl group can be optionally substituted with $R_2$.

10. A use of **Claim 6** wherein the $5HT_{1A}$ antagonist is 1-(4-indolyloxy)-3-cyclohexylamino-2-propanol or a pharmaceutically acceptable salt thereof.